# EUROPEAN PATENT APPLICATION

(11) **EP 2 305 709 A1**
(43) Date of publication of application: **06.04.2011**
(21) Application number: 10011650.8
(22) Date of filing: 09.04.1998
(51) Int. Cl.: C07K 16/18, C12N 15/00, A61K 48/00, A61P 25/28

(54) **Recombinant antibodies specific for beta-amyloid ends, DNA encoding and methods for use thereof**

(30) Priority: 09.04.1997 US 41850 P
(62) Divisional of application: 08011798.9
(71) Applicant: Intellect Neurosciences, Inc., New York, NY 10001 (US)
(72) Inventor: Chain, Daniel G., New York, NY 10003 (US)
(74) Representative: Grünecker, Kinkeldey, Stockmair & Schwanhäusser Anwaltssozietät

(57) **Abstract**

DNA encoding a recombinant antibody molecule end-specific for an amyloid-β peptide, pharmaceutical compositions thereof, and a method for preventing or inhibiting progression of Alzheimer's Disease by introducing such a DNA molecule into brain cells to express the recombinant antibody molecule and prevent the accumulation of amyloid- β peptides in the cerebrospinal fluid.

<110> Intellect Neurosciences, Inc.

<120> RECOMBINANT ANTIBODIES SPECIFIC FOR BETA-AMYLOID ENDS,
      DNA ENCODING AND METHODS OF USE THEREOF

<130> EP37487IIHV163pau

<140> not yet assigned
<141> herewith

<150> 08 011 798.9
<151> 2008-06-30

<150> 98 918 035.1
<151> 1998-04-09

<150> PCT/US98/06900
<151> 1998-04-09

<150> 60/041,850
<151> 1997-04-09

<160> 8

<170> PatentIn Ver. 2.0


<210> 1
<211> 59
<212> PRT
<213> Homo sapiens

<400> 1
Glu Val Lys Met Asp Ala Glu Phe Arg His Asp Ser Gly Tyr Glu Val
  1               5                  10                  15
His His Gln Lys Leu Val Phe Phe Ala Glu Asp Val Gly Ser Asn Lys
             20                  25                  30
Gly Ala Ile Ile Gly Leu Met Val Gly Gly Val Val Ile Ala Thr Val
         35                  40                  45
Ile Val Ile Thr Leu Val Met Leu Lys Lys Lys
     50                  55


<210> 2
<211> 6
<212> PRT
<213> Artificial Sequence

<220>
<223> HUMAN PEPTIDE WITH ARTIFICIAL TERMINAL CYSTEINE RESIDUE

<400> 2
Asp Ala Glu Phe Arg Cys
  1               5


<210> 3
<211> 8
<212> PRT
<213> Artificial Sequence

<220>
<223> HUMAN PEPTIDE WITH ARTIFICIAL TERMINAL CYSTEINE RESIDUE

<400> 3
Asp Ala Glu Phe Arg His Asp Cys
  1               5

<210> 4
<211> 8
<212> PRT
<213> Artificial Sequence

<220>
<223> HUMAN PEPTIDE WITH ARTIFICIAL TERMINAL CYSTEINE RESIDUE

<400> 4
Cys Leu Met Val Gly Gly Val Val
1               5


<210> 5
<211> 8
<212> PRT
<213> Artificial Sequence

<220>
<223> HUMAN PEPTIDE WITH ARTIFICIAL TERMINAL CYSTEINE RESIDUE

<400> 5
Cys Val Gly Gly Val Val Ile Ala
1               5


<210> 6
<211> 6
<212> PRT
<213> Homo sapiens

<400> 6
Asp Ala Glu Phe Arg His
1               5


<210> 7
<211> 13
<212> PRT
<213> Homo sapiens

<400> 7
Glu Ile Ser Glu Val Lys Met Asp Ala Glu Phe Arg His
1               5                   10


<210> 8
<211> 4
<212> PRT
<213> Homo sapiens

<400> 8
Glu Phe Arg His
1

## Description

### CROSS-REFERENCE TO RELATED APPLICATIONS

The present application claims priority under 35 USC 119(e) from U.S. provisional application no. 60/041,850, filed April 9, 1997, the entire contents of which are hereby incorporated by reference.

### BACKGROUND OF THE INVENTION

### Field of the Invention

The present invention relates to a method for preventing or inhibiting progression of Alzheimer's Disease through gene delivery to cells of the central nervous system. The present invention also relates to a recombinant DNA molecule containing a gene encoding a recombinant antibody molecule end-specific for an amyloid-β peptide operably-linked to a promoter capable of expressing a recombinant antibody in cells of the central nervous system, and pharmaceutical compositions thereof.

### Description of the Background Art

A major histopathological hallmark of Alzheimer's Disease (AD) is the presence of amyloid deposits within neuritic and diffuse plaques in the parenchyma of the amygdala, hippocampus and neocortex (Glenner and Wong, 1984; Masters et al., 1985; Sisodia and Price, 1995). Amyloid is a generic term that describes fibrillar aggregates that have a common β-pleated structure. These aggregates exhibit birefringent properties in the presence of Congo red and polarized light (Glenner and Wong, 1984). The diffuse plaque is thought to be relatively benign in contrast to the neuritic plaque which appears to be strongly correlated with reactive and degenerative processes (Dickson et al., 1988; Tagliavini et al., 1988; Yamaguchi et al., 1989; Yamaguchi et al., 1992). The principal component of neuritic plaques is a 42 amino acid residue amyloid-β (Aβ) protein (Miller et al., 1993; Roher et al., 1993) that is derived from the much larger β-amyloid precursor protein, βAPP (or APP) (Kang et al., 1987). Aβ 1-42 is produced less abundantly than the 1-40 Aβ peptide (Haass et al., 1992; Seubert et al., 1992), but the preferential deposition of Aβ1-42 results from the fact that this COOH-extended form is more insoluble than 1-40 Aβ and is more prone to aggregate and form anti-parallel β-pleated sheets (Joachim et al., 1989; Halverson et al., 1990; Barrow et al., 1992; Burdick et al., 1992; Fabian et al., 1994). Aβ1-42 can seed the aggregation of Aβ 1-40 (Jarrett and Lansbury 1993).

The APP gene was sequenced and found to be encoded on chromosome 21 (Kang et al., 1987). Expression of the APP gene generates several Aβ-containing isoforms of 695, 751 and 770 amino acids, with the latter two βAPP containing a domain that shares structural and functional homologies with Kunitz serine protease inhibitors (Kang et al., 1987; Kitaguchi et al., 1988; Ponte et al., 1988; Tanzi et al., 1988; Konig et al., 1992). The functions of βAPP in the nervous system remain to be defined, although there is increasing evidence that βAPP has a role in mediating adhesion and growth of neurons (Schubert et al., 1989; Saitoh et al., 1994; Saitoh and Roch, 1995) and possibly in a G protein-linked signal transduction pathway (Nishimoto et al., 1993). In cultured cells, βAPPs mature through the constitutive secretory pathway (Weidemann et al., 1989; Haass et al., 1992; Sisodia 1992) and some cell-surface-bound βAPPs are cleaved within the Aβ domain by an enzyme, designated α-secretase, (Esch et al., 1990), an event that precludes Aβ amyloidogenesis. Several studies have delineated two additional pathways of βAPP processing that are both amyloidogenic: first an endosomal/lysosomal pathway generates a complex set of βAPP-related membrane-bound fragments, some of which contain the entire Aβ sequence (Haass et al., 1992; Golde et al., 1992); and second, by mechanisms that are not fully understood, Aβ 1-40 is secreted into the conditioned medium and is present in cerebrospinal fluid *in vivo* (Haass et al., 1992; Seubert et al., 1992; Shoji et al., 1992; Busciglio et al., 1993). Lysosomal degradation is no longer thought to contribute significantly to the production of Aβ (Sisodia and Price, 1995). The proteolytic enzymes responsible for the cleavages at the NH₂ and COOH termini of Aβ termed β and γ, respectively, have not been identified. Until recently, it was generally believed that Aβ is generated by aberrant metabolism of the precursor. The presence, however, of Aβ in conditioned medium of a wide variety of cells in culture and in human cerebrospinal fluid indicate that Aβ is produced as a normal function of cells.

If amyloid deposition is a rate-limiting factor to produce AD, then all factors linked to the disease will either promote amyloid deposition or enhance the pathology that is provoked by amyloid. The likelihood of amyloid deposition is enhanced by trisonomy 21 (Down's syndrome) (Neve et al., 1988; Rumble et al., 1989), where there is an extra copy of the APP gene, by increased expression of APP, and by familial Alzheimer's Disease (FAD)-linked mutations (Van Broeckhoven et al., 1987; Chartier-Harlin et al., 1991; Goate et al., 1989; Goate et al., 1991; Murrell et al., 1991; Pericak-Vance et al., 1991; Schellenberg et al., 1992; Tanzi et al., 1992; Hendricks et al., 1992; Mullan et al., 1992). Some of these mutations are correlated with an increased total production of Aβ (Cai et al., 1993; Citron et al., 1992) or specific overproduction of the more fibrillogenic peptides (Wisniewski et al., 1991; Clements et al., 1993; Susuki et al., 1994) or increased expression of factors that induce fibril formation (Ma et al., 1994; Wisniewski et al., 1994). Collectively, these findings strongly favor the hypothesis that amyloid deposition is a critical element in the development of AD (Hardy 1992), but of course they do not preclude the possibility that other age-related changes associated with the disease, such as paired helical filaments, may develop in parallel rather than as a result of amyloid deposition and contribute to dementia independently.

The main focus of researchers and the principal aim of those associated with drug development for AD is to reduce the amount of Aβ deposits in the central nervous system (CNS). These activities fall into two general areas: factors affecting the production of Aβ, and factors affecting the formation of insoluble Aβ fibrils. A third therapeutic goal is to reduce inflammatory responses evoked by Aβ neurotoxicity.

With regards to the first, a major effort is underway to obtain a detailed understanding of how newly synthesized βAPP is processed for insertion into the plasma membrane and to identify the putative amyloidogenic secretases that have been assigned on the basis of sites for cleavage in the mature protein. From a pharmacological perspective, the most direct way of reducing the production of Aβ is through direct inhibition of either β or γ secretase. No specific inhibitors are currently available although a number of compounds have been shown to indirectly inhibit the activities. Bafilomycin, for example, inhibits Aβ production with an EC₅₀ of about 50 nM (Knops et al., 1995; Haass et al., 1995), most likely through its action as an inhibitor of vacuolar H*ATPase co-localized in vesicles with the Aβsecretase. Another compound, MDL28170, used at high concentrations appears to block the activity of γ secretase Higaki et al., 1995). It is generally hoped that the identification of the β or γ secretases might lead to the synthesis of specific protease inhibitors to block the formation of amyloidogenic peptides. It is not known, however, whether these enzymes are specific for βAPP or whether they perhaps have other important secretory functions. Similarly, problems of target and targeting specificity will be encountered through any attempt to interfere with signal transduction pathways that may determine whether processing of βAPP is directed through the amyloidogenic or non-amyloidogenic pathways. Moreover, these signal transduction mechanisms still need to be identified. In conclusion, present understanding of the complex and varied underlying molecular mechanisms leading to overproduction of Aβ offers little hope for selective targeting by pharmacological agents.

Given that neurotoxicity appears to be associated with β-pleated aggregates of Aβ, one therapeutic approach is to inhibit or retard Aβ aggregation. The advantage of this approach is that the intracellular mechanisms triggering the overproduction of Aβ or the effects induced intracellularly by Aβ need not be well understood. Various agents that bind to Aβ are capable of inhibiting Aβ neurotoxicity in vitro, for example, the Aβ-binding dye, Congo Red, completely inhibits Aβ-induced toxicity in cultured neurons (Yankner et al., 1995). Similarly, the antibiotic rifampacin also prevents Aβ aggregation and subsequent neurotoxicity (Tomiyama et al., 1994). Other compounds are under development as inhibitors of this process either by binding Aβ directly, e.g., hexadecyl-N-methylpiperidinium (HMP) bromide (Wood et al., 1996), or by preventing the interaction of Aβ with other molecules that contribute to the formation of Aβ deposition. An example of such a molecule is heparan sulfate or the heparan sulfate proteoglycan, perlecan, which has been identified in all amyloids and is implicated in the earliest stages of inflammation associated amyloid induction.

Heparan sulfate interacts with the Aβ peptide and imparts characteristic secondary and tertiary amyloid structural features. Recently, small molecule anionic sulfates have been shown to interfere with this reaction to prevent or arrest amyloidogenesis (Kisilevsky, 1995), although it is not evident whether these compounds will enter the CNS. A peptide based on the sequence of the perlecan-binding domain appears to inhibit the interaction between Aβ and perlecan, and the ability of Aβ-derived peptides to inhibit self-polymerization is being explored as a potential lead to developing therapeutic treatments for AD. The effectiveness of these compounds in vitro, however, is likely to be modest for a number of reasons, most notably the need for chronic penetration of the blood brain barrier.

As another means of inhibiting or retarding Aβ aggregation, WO 96/25435 discloses the potential for using a monoclonal antibody, which is end-specific for the free C-terminus of the Aβ 1-42 peptide, but not for the Aβ 1-43 peptide, in preventing the aggregation of Aβ 1-42. While the administration of such an Aβ end-specific monoclonal antibody is further disclosed to interact with the free C-terminal residue of Aβ 1-42, thereby interfering with and disrupting aggregation that may be pathogenic in AD, there is no specific disclosure on how these Aβ C-terminal-specific monoclonal antibodies would be used therapeutically. Although direct or indirect manipulation of Aβ peptide aggregation appears to be an attractive therapeutic strategy, a possible disadvantage of this general approach may be that pharmacological compounds of this class will need to be administered over a long period of time, and may accumulate and become highly toxic in the brain tissue.

An alternative to a peptide-based approach is to elucidate the cellular mechanism of Aβ neurotoxicity and develop therapeutics aimed at those cellular targets. The focus has been on controlling calcium dysfunction of free radical mediated neuronal damage. It has been postulated that Aβ binds to RAGE (the receptor for advanced glycation end-products) on the cell surface, thereby triggering reactions that could generate cytotoxic oxidizing stimuli (Yan et al., 1996). Blocking access of Aβ to the cell surface binding site(s) might retard progression of neuronal damage in AD. To date there are no specific pharmacological agents for blocking Aβ-induced neurotoxicity.

In addition to therapeutic approaches through the direct administration of pharmacologically active agents, WO 89/01975 discloses a method of transplanting glial cells (actively secreting cells derived from within the brain), which have been transformed to express and secrete recombinant polymeric anti-acetylcholinesterase antibodies of the IgM class. It is predicted in the disclosure of WO 89/01975 that the antibody secreted by the transformed cells transplanted into the brain of a person suffering from Alzheimer's Disease may then alleviate or abolish the symptoms of the disease. This is a gene therapeutic approach arising from the observation that cells of the central nervous system are very efficient in the secretion of antibodies (Cattaneo and Neuberger, 1987). Piccioli et al., 1991 and 1995, later demonstrated the ectopic neuronal expression of recombinant antibodies from the promoter of the neuronal vgf gene in a tissue-specific and developmentally regulated manner. Thus, non-lymphoid cells, and in particular, neuronal cells were found to be capable of secreting functional immunoglobulins.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention relates to a novel method for preventing the onset of Alzheimer's Disease or for inhibiting progression of Alzheimer's Disease through the stable expression in the brain of recombinant antibodies end-specific for amyloid-β peptides. These ectopically expressed recombinant antibody molecules, which are end-specific for the N-terminus or C-terminus of amyloid-β peptides, prevent the accumulation of amyloid-β peptides in the extracellular space, interstitial fluid and cerebrospinal fluid and the aggregation of such peptides into amyloid deposits in the brain. Given the many possible mechanisms that might contribute to the production of amyloid-β, coupled with the tremendous diversity of interactions of Aβ with the cell surface and extracellular Aβ-binding molecules capable of bringing about chronic neurotoxicity, the present method is directed to preventing the accumulation of Aβ peptides in the extracellular milieu of affected neurons as the focal point of this heterogeneous pathological cascade. The present invention also avoids the problems associated with the repeated administration of pharmacological agents that requires chronic penetration of the blood brain barrier.

It is therefore an object of the invention to overcome the deficiencies in the prior art by providing a novel method for preventing or inhibiting the progression of Alzheimer's Disease.

Another object of the invention is to provide a method whereby cells of the nervous system are conferred with the ability to ectopically express recombinant antibody molecules in the brain, which molecules are end-specific for the N-terminus or C-terminus of amyloid-β peptides, to prevent the accumulation of amyloid-β peptides in the extracellular space, interstitial fluid and cerebrospinal fluid and the aggregation of such peptides into amyloid deposits in the brain.

A further object of the invention is to provide a method for preventing or inhibiting the progression of Alzheimer's Disease by also inhibiting the interaction of amyloid-β peptides mediating amyloid-β induced neurotoxicity and inhibiting the amyloid-β induced complement activation and cytokine release involved in the inflammatory process associated with Alzheimer's Disease.

Still another object of the invention is to provide a recombinant DNA molecule, containing a gene encoding a recombinant antibody molecule end-specific for the N-terminus or the C-terminus of an amyloid-β peptide and operably-linked to a promoter which is expressed in the central nervous system.

Yet another object of the invention is to provide a vector for introducing the recombinant DNA molecule into cells of the central nervous system.

Still yet another object of the invention is to provide a pharmaceutical composition for preventing or inhibiting the progression of Alzheimer's Disease.

The inventions provides a method for preventing or inhibiting progression of Alzheimer's Disease, comprising the step of administering a composition comprising a recombinant DNA molecule, containing a gene encoding a recombinant antibody molecule end-specific for the N-terminus or the C-terminus of an amyloid-β peptide, operably-linked to a promoter which is expressed in the central nervous system, in association with a means for gene delivery, to a patient in need thereof to prevent the accumulation of amyloid-β peptides and the aggregation of peptides which form amyloid deposits in the brain.

In a second aspect of the invention, the composition is administered by injection intravenously, intra-arterially, intracranially, or intracephalically.

In a third aspect of the invention, the amyloid-β peptide is selected from the group consisting of amyloid-β peptides having the amino acid sequence of residues 5-44 of SEQ ID NO:1, residues 5-46 of SEQ ID NO:1, residues 5-47 of SEQ ID NO:1, and mixtures thereof.

In a fourth aspect of the invention, the recombinant antibody molecule is end-specific for the N-terminus of the amyloid-β peptide.

In a fifth aspect of the invention, the recombinant antibody molecule is end-specific for the C-terminus of the amyloid-β peptide.

In a sixth aspect of the invention, the promoter operably-linked to the gene encoding a recombinant antibody molecule is a βAPP promoter.

In a seventh aspect of the invention, the means for gene delivery in association with the recombinant DNA molecule comprises a viral vector. Preferably, in an eighth aspect, the viral vector is adeno-associated vector (AAV). In a further preferred ninth aspect, the means for gene delivery further comprises cationic lipids or cationic liposomes.

In a tenth aspect the means for gene delivery in association with the recombinant DNA molecule comprises cationic lipids or cationic liposomes.

In an eleventh aspect, the means for gene delivery in association with the recombinant DNA molecule comprises a ligand capable of binding to a cell surface receptor. Preferably, in an eleventh aspect, the ligand is biotin.

In a thirteenth aspect, the recombinant antibody molecule is a single chain variable region fragment.

The invention also provides an antibody end-specific for the N-terminus of an amyloid β-peptide.

The invention also provides a recombinant DNA molecule, comprising a gene encoding a recombinant antibody molecule end-specific for the N-terminus or the C-terminus of anamyloid-β peptide and a promoter operably linked to said gene, wherein said promoter is capable of expressing said recombinant antibody molecules in brain cells. Preferably, said promoter is a βAPP promoter. The invention also provides a vector comprising the recombinant DNA molecule and a host cell transformed with said vector.

In a further embodiment, the invention provides a pharmaceutical composition for preventing or inhibiting progression of Alzheimer's Disease, comprising the aforementioned recombinant DNA molecule in association with a means for gene delivery, and a pharmaceutically acceptable excipient. Preferably, the means for gene delivery is selected from the group consisting of viral vectors, cationic lipids, cationic liposomes, ligands capable of binding to a cell surface receptor, and combinations thereof. Preferably, said gene encodes a recombinant antibody molecule end-specific for the N-terminus or the C-terminus of an amyloid-β peptide.

### BRIEF DESCRIPTION OF THE DRAWINGS

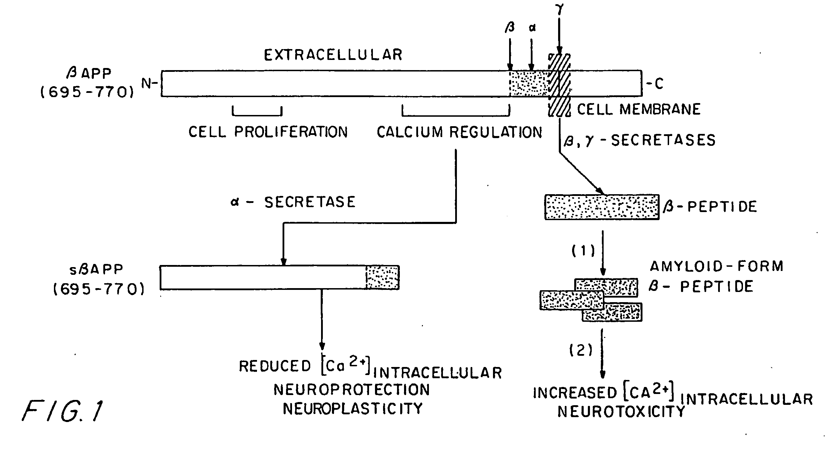

Figure 1 shows a schematic representation of the β-amyloid precursor protein (βAPP) and the products of α, β, and γ-secretase cleavage. The general locations of various domains are indicated along with the cleavage sites (α, β, γ) for secretases. Figure 1 also schematically shows that the stable expression and secretion of ectopic Aβ-end-specific antibodies in the CNS inhibits (1) the accumulation of Aβ peptides and (2) the neurotoxic consequences of amyloid deposition without affecting the biological functions of the soluble β-amyloid precursor protein.

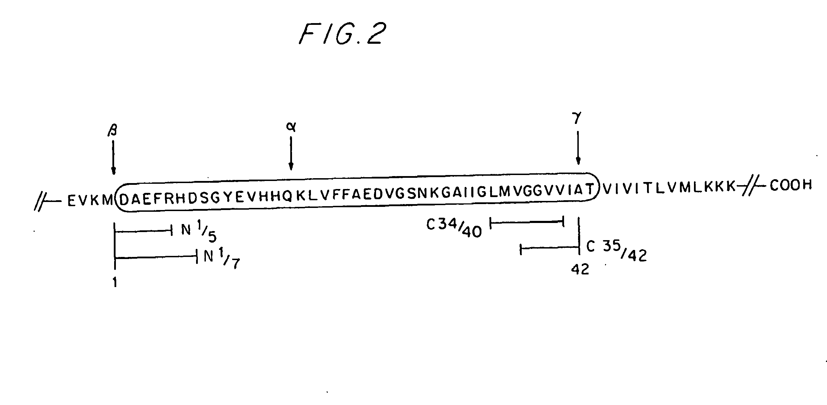

Figure 2 shows the amino acid sequence (SEQ ID NO:1) of the region in βAPP from which β-amyloid peptides (Aβ) are derived. The arrows indicate the α-, β- or γ-secretase cleavage sites, and the amino acid residues corresponding to the synthetic peptides to be used as immunogens are indicated underneath the sequence by line segments.

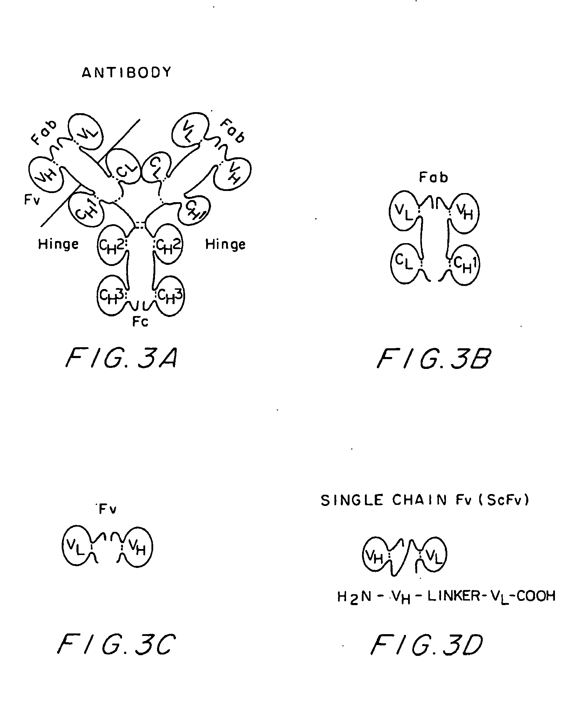

Figures 3A-3D schematically show the structure of a whole antibody (Fig. 3A) with the variable domain of heavy (V_{H}) and light (V_{L}) chains and the constant domain(s) of light (C_{L}) and heavy (C_{H}1, C_{H}2, C_{H}3) chains, a Fab fragment (Fig. 3B), a Fv fragment (Fig. 3C), and a single chain Fv fragment (scFv) (Fig. 3D). The Fab fragment shown in Fig. 3B consists of a variable domain of heavy V_{H} and light V_{L} chain and the first constant domain (C_{H}1 and C_{L}) joined by a disulfide bridge. The Fv fragment shown in Fig. 3C represents the antigen binding portion of an antibody formed by a non-covalently linked variable region complex (V_{H}-V_{L}), whereas the single chain Fv shown in Fig. 3D joins the variable heavy V_{H} with the variable light V_{L} chain via a peptide linker.

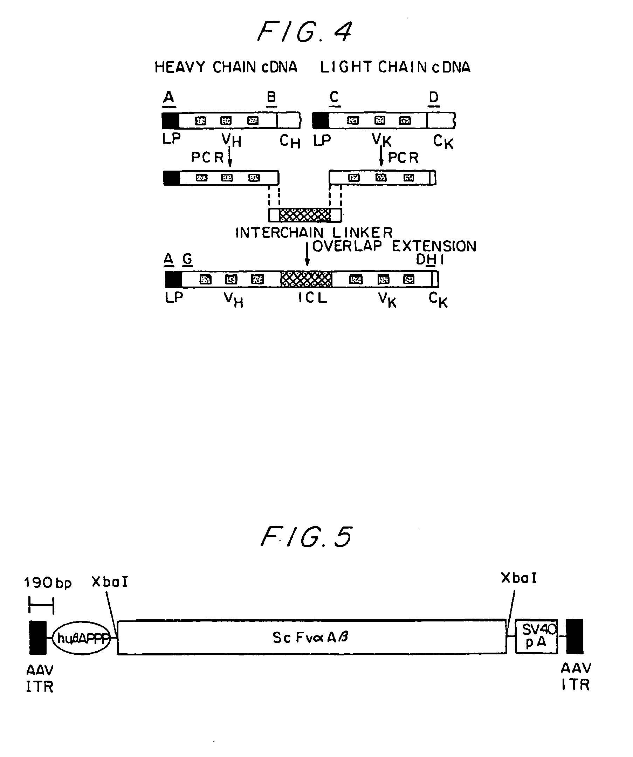

Figure 4 schematically shows the construction of a scFv antibody by cloning the variable region of an end-specific anti-Aβ monoclonal antibody using the PCR amplification technique with primers A, B, C and D, and then joining together the variable heavy V_{L} chain and the variable light V_{L} chain with an interchain peptide linker (ICL). Then shaded area represents hypervariable regions of the antigen binding site and LP designates the leader peptide of the heavy and light chains.

Figure 5 shows a schematic representation of the AAV ScFvαAβ vector with the inverted terminal repeats (ITR), human βAPP promoter (HuβAPPP), SV40 polyadenylation signal (SV40pA) indicated. The plasmid backbone is pSSV9.

### DETAILED DESCRIPTION OF THE INVENTION

The novel DNA molecules of the present invention contain a gene encoding a recombinant antibody molecule end-specific for the N-terminus or the C-terminus of an Aβ peptide. Such a recombinant antibody molecule discriminates between an Aβ peptide and the β-amyloid protein precursor from which it is proteolytically derived, and is also referred to throughout the present specification as an "antisenilin". By "antisenilin" is meant a molecule which binds specifically to a terminus/end of an Aβ peptide to slow down or prevent the accumulation of amyloid-β peptides in the extracellular space, interstitial fluid and cerebrospinal fluid and the aggregation into senile amyloid deposits or plaques and to block the interaction of Aβ peptides with other molecules that contribute to the neurotoxocity of Aβ.

The method for preventing or inhibiting the progression of Alzheimer's Disease in accordance with the present invention, involves delivering the gene encoding the antisenilin molecule into brain cells where antisenilins are then stably expressed and secreted into the extracellular space, interstitial fluid and cerebrospinal fluid. The secretion of antisenilins into the extracellular space, interstitial fluid and cerebrospinal fluid, where soluble Aβ peptides are present, promotes the formation of soluble antisenilin-Aβ complexes. These soluble antisenilin-Aβ complexes are cleared from the central nervous system by drainage of the extracellular space, interstitial fluid and cerebrospinal fluid into the general blood circulation through the arachnoid villi of the superior sagittal sinus. In this manner, soluble Aβ peptides are prevented from accumulating in the extracellular space, interstitial fluid and cerebrospinal fluid to form amyloid deposits and/or to induce neurotoxicity (Fig. 1). Furthermore, clearance of soluble amyloid-β peptides in accordance with the present invention is expected to reduce the inflammatory process observed in Alzheimer's Disease by inhibiting, for example, amyloid-β-induced complement activation and cytokine release, and block also the interaction of Aβ with cell surface receptors such as the RAGE receptor.

The composition of the present invention includes a recombinant DNA molecule containing an antisenilin gene in association with a means for gene delivery where this composition may be for use as a medicament for preventing or inhibiting the progression of Alzheimer's Disease.

As shown in Fig. 1 (see Schehr, 1994), and discussed in the Background Art section, the β-amyloid protein precursor (βAPP) is believed also to serve as a precursor for a proteolytic product, soluble β-amyloid protein precursor (sβAPP), thought to have growth promoting and neuroprotective functions. It will be readily appreciated by those of skill in the art that the stable expression of antisenilins in the central nervous system will not interfere with the normal biological functions of βAPP that are not associated with the formation of Aβ peptides. In the novel recombinant DNA molecules of the present invention, the gene encoding an antisenilin molecule contains at least the nucleotide sequences which encode the antigen-binding domain of an end-specific monoclonal antibody molecule. Thus, the antisenilin molecule, which is a recombinant antibody molecule containing the antigen-binding portion of a monoclonal antibody, is intended to encompass a chimeric or humanized immunoglobulin molecule of any isotype, as well as a single-chain antibody.

Chimeric antibodies are understood to be molecules, different portions of which are derived from different animal species, such as those having a variable region derived from a mouse monoclonal antibody and a human immunoglobulin constant region. Chimeric antibodies and methods for their production are well known in the art. For example, the DNA encoding the variable region of the antibody can be inserted into or joined with DNA encoding other antibodies to produce chimeric antibodies (U.S. patent 4,816,567; Orlandi et al., 1989).

Single-chain antibodies as antisenilins can also be produced according to the present invention. These single chain antibodies can be single chain composite polypeptides having end-specific Aβ peptide binding capability and comprising a pair of amino acid sequences homologous or analogous to the variable regions of an immunoglobulin light and heavy chain (linked V_{H}-V_{L} or single chain Fv). Both V_{H} and V_{L} may copy natural monoclonal antibody sequences, or one or both of the chains may comprise a CDR-FR construct of the type described in U.S. Patent 5,091,513. The separate polypeptides analogous to the variable regions of the light and heavy chains are held together by a peptide linker. Methods of production of such single chain antibodies, e.g., single chain Fv (scFv), particularly where the DNA encoding the polypeptide structures of the V_{H} and V_{L} chains are characterized or can be readily ascertained by sequence analysis, may be accomplished in accordance with the methods described, for example, in U.S. Patent 4,946,778, U.S. Patent 5,091,513, U.S. Patent 5,096,815, Biocca et al., 1993, Duan et al., 1994, Mhashilkar et al., 1995, Marasco et al., 1993, and Richardson et al., 1995. Figures 3A-3D (from Biocca et al., 1995) schematically show an intact antibody (Fig. 3A), a Fab fragment (Fig. 3B), a Fv fragment consisting of a non-covalently linked variable region complex (V_{H}-V_{L} (Fig. 3C), and a single chain Fv antibody (Fig. 3D).

In constructing the recombinant gene encoding the antisenilin molecule, a hybridoma producing a monoclonal antibody end-specific for the N-terminus or C-terminus of an amyloid-β peptide is first obtained, where an end-specific antibody is defined as an antibody which uniquely recognizes the free N-terminus or the free C-terminus of a peptide and which can further discriminate between the peptide and the precursor from which it is proteolytically derived. The design of immunogenic peptides for use in immunization and the generation of monoclonal antibody producing hybridomas is based on similar peptides that have been previously used by several laboratories to generate monoclonal antibodies that uniquely recognize the free amino or carboxy-terminal of Aβ (Harrington et al., 1993; Iwatsubo et al., 1994; Konig et al., 1996; Murphy et al., 1994; Gravina et al., 1995). While peptides of longer lengths have in some instances been used successfully to generate Aβ end-specific antibodies, Saido and co-workers (1993; 1994) established that there is a length of five amino acids for any given peptide which ensures that the specific free amino group at the N-terminus constitutes an essential part of the epitope recognized by the new antibody. Thus, a monoclonal antibody generated against an immunogenic peptide is evaluated for the selectivity of the antibody in its recognition of a free Nor C-terminus of an Aβ peptide. A competitive inhibition assay, using Enzyme-Linked Immunosorbant Assay (ELISA) or immunoprecipitation with peptides corresponding to different regions of Aβ and the region immediately preceding the β-secretase cleavage site in the extracellular domain of βAPP, can determine the selectivity of the monoclonal antibody. When clearance of the amyloid peptides involved in the pathogenesis of Alzheimer's Disease, i.e., Aβ1-40 (corresponding to residues 5-44 of SEQ ID NO:1), Aβ1-42 (corresponding to residues 5-46 of SEQ ID NO:1), and Aβ1-43 (corresponding to residues 5-47 of SEQ ID NO:1), is the major goal, then the monoclonal antibody is preferably end-specific for the N-terminus that is common to these Aβ peptides. In other cases, however, such as when used to treat a patient following the onset of Alzheimer's Disease, it may be preferable to select an antibody that will also interfere with the ability of Aβ peptides to seed aggregation or to interact with other molecules that either contribute to the seeding of Aβ deposition or mediate Aβ-induced cytotoxic effects. Immunogenic peptides of varying lengths, which incorporate either the free N-terminus or free C-terminus, are synthesized to allow for generating end-specific anti-Aβ antibodies and the recombinant DNA encoding for a recombinant Aβ end-specific antibody (antisenilin) used in a pharmaceutical composition for this type of selective application in preventing or inhibiting the progression of Alzheimer's Disease.

Those of skill in the art will appreciate that a cysteine residue can be added to the end of the above immunogenic peptides opposite from the end corresponding to the free N-terminus or the free C-terminus of Aβ peptides to facilitate coupling to a carrier protein. Keyhole limpet hemocyanin (KLH), ovalbumin and bovine serum albumin (BSA) are non-limiting examples of proteins that can be used as carriers for immunogens. The presence of an N-terminal or C-terminal cysteine residue on the synthetic immunogen peptides provides a free sulfhydryl group for covalent coupling to a maleimide-activated protein. A heterobifunctional reagent, such as an N-maleimido-6-aminocaproyl ester or a m-maleimidobenzoyl-N-hydroxysuccinimide ester (MBS), is used to covalently couple the synthetic immunogenic peptide to the carrier protein (see for example, Hartlow, E. et al., Antibodies: A Laboratory Manual, Cold Spring Harbor Laboratory Press, Cold Spring Harbor, N.Y. 1988). Commercial kits are also readily available for use in coupling peptide antigens to maleimide-activated large carrier proteins.

Monoclonal antibodies may be obtained by methods known to those skilled in the art. See, for example Kohler and Milstein, 1975; U.S. Patent No. 4,376,110; Ausubel et al., eds., Current Protocols in Molecular Biology, Greene Publishing Assoc. and Wiley Interscience, N.Y., (1987, 1992); and Harlow et al., supra; Colligan et al., eds., Current Protocols in Immunology, Greene Publishing Assoc. and Wiley Interscience, N.Y., (1992-1997), the contents of which references are incorporated entirely herein by reference.

Once monoclonal antibodies are generated, the selectivity and binding affinity (Kd) can be evaluated by ELISA, and in vitro bioassays can be performed on the antibodies to test for the efficacy of the Aβ end-specific monoclonal antibodies in blocking Aβ aggregation and Aβ-induced cytotoxicity as described below in Example 1. Preferably, these monoclonal antibodies have not only a selectivity that is end-specific for specific Aβ peptides, but also have a high binding affinity. It is intended that the DNA encoding any antibody that is end-specific for the N-terminus or C-terminus of Aβ peptides and exhibits efficacy in blocking Aβ aggregation and Aβ induced cytotoxicity as described in Example 1 can be used in generating the recombinant antisenilin-encoding DNA molecules for use according to the present invention. For instance, the C-terminal end-specific monoclonal antibodies disclosed in WO 96/25435 may be used to obtain the recombinant antisenilin-encoding DNA molecules according to the present invention.

Messenger RNA (mRNA) may then be isolated from hybridomas producing Aβ end-specific monoclonal antibodies determined to be selective for the free N-terminus or free C-terminus of Aβ peptides. From the isolated hybridoma mRNA, cDNA is synthesized and the nucleotide sequence encoding the variable domains of the Aβ end-specific monoclonal antibody may then be cloned using the polymerase chain reaction (PCR) with primers based on the conserved sequences at each end of the nucleotide sequences encoding the V domains of immunoglobulin heavy chain (V_{H}) and light-chain (V_{L}). The presence of restrictions sites incorporated into the sequence of the PCR primers facilitates the cloning of PCR amplified products encoding the variable region of the appropriate chain.

A recombinant gene encoding a recombinant single chain Fv antibody molecule is constructed, for example, by joining nucleotide sequences encoding the V_{H} and V_{L} domains with a nucleotide sequence encoding a peptide interchain linker (Biocca et al., 1993; Duan et al., 1994; Mhashilkar et al., 1995; Marasco et al., 1993; Richardson et al., 1995; U.S. Patent 4,946,778; U.S. Patent 5,091,513, U.S. Patent 5,096,815) or by inserting the variable domain-encoding nucleotide sequences to replace the corresponding sequences encoding the variable domain in a human immunoglobulin gene to thereby encode for a recombinant chimeric antibody (Orlandi et al., 1989; U.S. Patent 4,816,567).

Standard reference works setting forth the general principles of recombinant DNA technology include Ausubel et al., eds., Current Protocols In Molecular Biology, Green Publishing Assoc. and Wiley Interscience, N.Y. (1987-1997), Watson et al., Molecular Biology of the Gene, Volumes I and II, The Benjamin/Cummings Publishing Company, Inc., publisher, Menlo Park, CA (1987); Darnell et al., Molecular Cell Biology, Scientific American Books, Inc., publisher, New York, N.Y. (1986); Lewin, Genes II, John Wiley & Sons, publishers, New York, N.Y. (1985); Old et al., Principles of Gene Manipulation: An Introduction to Genetic Engineering, 2d edition, University of California Press, publisher, Berkeley, CA (1981); Maniatis et al., Molecular Cloning: A Laboratory Manual, Cold Spring Harbor Laboratory, Cold Spring Harbor, NY (1989); and Berger et al., Guide to Molecular Cloning Techniques, Methods of Enzymology vo. 152, 1987, Academic Press, Inc. San Diego, CA. These references are hereby incorporated by reference.

The recombinant DNA molecule according to the present invention, which contains a recombinant antibody (antisenilin) gene, preferably also contains a promoter operably linked to the recombinant antisenilin gene and capable of expressing the antisenilin molecule in brain cells. It will also be appreciated that, in order to facilitate the secretion of the antisenilin molecule from transformed cells expressing antisenilin, a leader or signal peptide at the N-terminus is also provided.

A DNA molecule is said to be "capable of expressing" a polypeptide, such as the antisenilin molecule, if it contains nucleotide sequences which contain transcriptional and translational regulatory information, and such sequences are "operably linked" to nucleotide sequences which encode the polypeptide. An operable linkage is a linkage in which the regulatory DNA sequences and the DNA sequence sought to be expressed are connected in such a way as to permit gene expression. The regulatory regions needed for gene expression in general include a promoter region as well as the DNA sequences which, when transcribed into RNA, will signal the initiation of protein synthesis. Such regions will normally include those 5'-non-coding sequences involved with initiation of transcription and translation.

A promoter region would be operably linked to a DNA sequence if the promoter were capable of effecting transcription of that DNA sequence. As used herein, a "promoter sequence" is the sequence of the promoter which is found on the DNA and is transcribed by the RNA polymerase. Thus, to express antisenilins, transcriptional and translational signals recognized by the host cell are necessary.

The present method for preventing or inhibiting the progression of Alzheimer's Disease involves administering to a patient in need thereof a composition comprising a recombinant DNA molecule in association with means for gene delivery into cells of the central nervous system. The recombinant DNA molecule carries a gene encoding an antisenilin molecule operably-linked to a promoter where this operable linkage enables the expression of antisenilin molecules in the brain. The promoter is preferably a promoter which would follow the expression pattern of βAPP with the highest level of expression in the hippocampus and cerebral cortex where amyloid deposition is most prevalent in Alzheimer's Disease. As a non-limiting example of a preferred promoter operably linked to the antisenilin gene, the thymidine kinase (Thyl) promoter has been shown to drive the expression of βAPP in a region-specific manner that mimics the natural expression of βAPP in the brain (Andra et al., 1996). Synapsin I promoter-based chimeric transgenes have been used to target expression of βAPP in the CA subfields of the hippocampus and in the piriform cortex in brains of transgenic mice (Howland et al., 1995). A high level of βAPP expression has been achieved in brain cortex of transgenic mice using a prion protein promoter (Hsiao et al., 1996). A number of advantages would be provided by using the βAPP gene promoter to express the antisenilin gene. In particular, the antisenilin gene under the control of the βAPP promoter would have identical anatomical and physiological patterns of expression as the βAPP gene. The human βAPP promoter has been characterized by a number of groups (e.g. Salbaum et al., 1988; La Fauci et al., 1989; Wirak et al., 1991; Lahiri and Nall, 1995). The promoter has several regulatory domains including a heat-shock element and consensus sequences for the binding of transcription factors. Thus, expression of antisenilins under the control of the βAPP gene can be enhanced as necessary in specific regions of the brain by applying any of a number of inducing agents, for example, growth factors, retinoic acid, and interleukin-1. A preproenkephalin promoter has also been reported to yield region-specific and long term expression in an adult rat brain after direct *in vivo* gene transfer (Kaplitt et al., 1994).

In order to facilitate the introduction of a recombinant DNA molecule carrying an antisenilin gene operably-linked to a promoter into cells of the central nervous system, a number of different means for gene delivery can be used in association with the recombinant DNA molecule. The term "means for gene delivery" is meant to include any technique suitable for delivery of DNA molecules across the blood brain barrier and/or for transmembrane delivery across cell membranes. Non-limiting examples of the means for gene delivery are viral vectors (e.g., adeno-associated virus-based vectors), lipids/liposomes, ligands for cell surface receptors, etc.

The recombinant DNA molecule carrying the antisenilin gene is associated with the means for gene delivery where such association is intended to encompass, for example, the situation in which the means for gene delivery in a viral vector and the antisenilin gene is incorporated in the DNA of the viral vector or packaged in the viral particle; the situation in which the means for gene delivery is a liposome and the antisenilin gene is complexed therewith; the situation in which the means for gene delivery is a ligand for a cell surface receptor and the antisenilin gene is conjugated or otherwise bound thereto; etc. Thus, "in association with" includes incorporating or packaging in, complexing with, conjugating or binding to, and any other manner of associating the antisenilin gene with the means for gene delivery. It will be appreciated that the recombinant DNA molecule may be in association with more than one means for gene delivery, particularly where the recombinant DNA molecule is to be delivered across both the blood brain barrier and the cell membrane of brain cells.

Adeno-associated virus (AAV) was initially isolated as a tissue culture contaminant and was later found as a non-pathogenic coinfecting agent during an adenovirus outbreak in children (Blacklow et al., 1968). It is a single-stranded DNA virus of the parvovirus group with a 4.7 kb genome. As one of the smallest human DNA viruses, AAV requires coinfection with a helper virus, usually an adenovirus or herpesvirus, for efficient replication in order to complete its life cycle (Carter, 1990). In the absence of helper virus infection, AAV becomes latent and stably integrates at high frequency, often at a specific site on chromosome 19 (Kotin et al., 1990; 1991; 1992; Samulski et al., 1991). The AAV genome has been sequenced and it was discovered that the sole sequence needed for integration of an AAV vector is in the terminal 145 nucleotide inverted terminal repeats (ITR), thus making the cloning capacity nearly 4.7 kb (Muzyczka, 1992). Due to the non-pathogenic nature of the virus, its broad host cell range, and its ability to take advantage of a natural mechanism for high frequency integration, AAV is particularly suitable as a vector for gene delivery/transfer into cells. Moreover, while conventional retroviruses have a requirement for genomic DNA synthesis, AAV vectors have a unique ability to introduce foreign genes into non-dividing or quiescent cells. These characteristics are being increasingly exploited for gene expression in the mammalian brain, and several genes related to Alzheimer's Disease have been expressed in the brain using AAV vectors (Makimura et al., 1996). Recent studies by Du et al., 1996, indicate that AAV vectors can efficiently transduce and stably express a foreign gene, e.g., *lacZ*, in post-mitotic human neurons. The expression of foreign genes in neuronal cells has also been reported using liposome-mediated transfection with AAV-derived plasmids (Meyer et al., 1995; Wu et al., 1994, 1995).

Low et al., U.S. Patent 5,108,921, reviews available methods for transmembrane delivery of molecules such as proteins and nucleic acids by the mechanism of receptor mediated endocytotic activity. These receptor systems include those recognizing galactose, mannose, mannose-6-phosphate, transferrin, asialoglycoprotein, transcobalamin (vitamin B₁₂), α-2 macroglobulins, insulin and other peptide growth factors such epidermal growth factor (EGF). Low et al. also teaches that nutrient receptors, such as receptors for biotin and folate, can be advantageously used to enhance transport across the cell membrane due to the location and multiplicity of biotin and folate receptors on the membrane surfaces of most cells, and the associated receptor mediated transmembrane transport processes. Thus, a complex formed between a compound to be delivered into the cytoplasm and a ligand, such as biotin or folate, is contacted with a cell membrane bearing biotin or folate receptors to initiate the receptor mediated trans-membrane transport mechanism and thereby permit entry of the desired compound into the cell.

A biotin ligand can be attached to a DNA molecule, for example, by incorporating commercially available biotinylated deoxynucleotide triphosphates, e.g., biotin-14-dATP or biotin-14-dCTP from Life Technologies, Inc., Gaithersburg, MD, using terminal deoxynucleotidyl transferase (Karger, B.D., 1989). Biotin-14-dATP is a dATP analog with biotin attached at the 6-position of the purine base by a 14-atom linker and biotin-14-dCTP is a dCTP analog with biotin attached at the N⁴-position of the pyrimidine base also by a 14-atom linker.

Whether incorporated into a viral-based or plasmid vector for packaging into a virus, attached to a neural receptor-binding ligand molecule, complexed with cationic lipids or cationic liposomes, or in association with other suitable means for gene delivery, the recombinant DNA molecule encoding an antisenilin gene operably linked to a promoter is administered to a subject by injection. Stereotactic microinjection into different brain regions through use of established coordinates can be used to deliver the viral packaged or ligand-bound recombinant DNA molecule directly into the extratellular environment, e.g., cerebrospinal fluid, surrounding brain cells for subsequent transmembrane delivery into the cells themselves.

As direct injection into the brain is an invasive procedure, it is preferred that the viral packaged or ligand-bound recombinant DNA molecule be administered by intravenous or intra-arterial injection. The viral packaged or ligand-bound recombinant DNA can further be in association with other means for gene delivery, such as to effect gene delivery across the blood-brain barrier into the central nervous system. Zhu et al., 1993, demonstrated that cationic lipid-plasmid DNA complexes can be delivered systemically to all tissues including the brain. Recently, it has also been shown that intra-arterially administered cationic liposomes containing the thymidine kinase gene was successful in a rat model of brain tumor where regression was achieved without apparent toxicity or histological damage (Laine et al., 1995). Gene delivery by liposomes is well covered in the scientific literature and in patent publications, and extensively reviewed by Lasic, D.D., In:Liposomes in Gene Delivery, CRC Press, Boca Raton, Florida, 1997, which is hereby incorporated entirely by reference.

Once delivered to the brain, the viral packaged recombinant DNA molecule, either ligand-bound or in association with another suitable means for gene delivery, transforms brain cells, which subsequently express antisenilin molecules (recombinant antibody molecules end-specific for Aβ peptides) and secrete the expressed antisenilins into the extracellular space, interstitial fluid and cerebrospinal fluid. The secreted antisenilins then form a soluble complex with Aβ peptide to which they are end-specific in the extracellular space, interstitial fluid and cerebrospinal fluid. These soluble antisenilin-Aβ peptide complexes prevent the aggregation of Aβ peptides into amyloid deposits and prevent Aβ-induced neurotoxicity by clearing Aβ peptides from the central nervous system through drainage of the extracellular space, interstitial fluid and cerebrospinal fluid into the general blood circulation where they will be eliminated by protease digestion. Accordingly, the accumulation of newly-secreted soluble Aβ peptides responsible for amyloid deposition and Aβ-induced neurotoxicity is prevented.

while the present method for preventing or inhibiting the progression of Alzheimer's Disease is intended to be primarily used for patients with a clear genetic disposition to developing Alzheimer's Disease, it can also be used prophylactically to "immunize" the population in general against the occurrence of such a prevalent and debilitating disease. The preferred route of administration is intravenous or intra-arterial. However, despite the invasiveness of microinjection directly into regions of the brain, this route of administration is intended to be within the scope of the invention. In particular, patients having Down's Syndrome or familial Alzheimer's Disease-linked mutations who are expected to develop Alzheimer's Disease due their predisposition or patients who already suffer from Alzheimer's Disease can be treated by direct microinjection into the brain. The benefit of this treatment is expected to outweigh the risks of an invasive technique such as injection into the brain.

The recombinant DNA molecule which contains an antisenilin gene in association with a means for gene delivery may be used in the preparation or manufacture of a medicament/pharmaceutical composition. The pharmaceutical compositions contain an amount of the recombinant DNA molecule effective to achieve its intended purpose. For instance, when the means for gene delivery is a viral vector, such as an AAV vector, a suitable dosage of viral particles in a pharmaceutical composition to be stereotactically microinjected into different locations in the brain is in the range of about 5x10⁴ to 1x10¹¹ particles. When a ligand, such as biotin, is used as the means for gene delivery by administration directly into the brain, ligand-bound DNA molecules in the range of about 0.5 to 100 µg are suitably used. For such ligand bound DNA molecules, it is preferred that the DNA molecules are condensed beforehand to protect these molecules in the extracellular milieu of cells within the central nervous system. Pharmaceutical compositions and dosages of DNA molecules complexed with cationic lipids or cationic liposomes are discussed in Lasic, 1997, supra. Furthermore, the pharmaceutical compositions may contain suitable pharmaceutically acceptable excipients, such as are well-known in the art.

Having now generally described the invention, the same will be more readily understood through reference to the following prophetic example, which is provided by way of illustration and is not intended to be limiting of the present invention.

### EXAMPLE 1

The strategy and the protocols for use in developing recombinant DNA molecules containing a gene encoding a recombinant antisenilin antibody molecule end-specific for an amyloid-β peptide are described below.

### MONOCLONAL Aβ END-SPECIFIC ANTIBODY PRODUCTION

### Immunogen peptide synthesis

Several peptides of varying lengths incorporating either the free N-terminus or free C-terminus are prepared using an Applied Biosystems Peptide Synthesizer (430A). The synthetic peptides are purified by HPLC and characterized using both amino acid composition and NH₂-terminal micro sequence analyses.

### Peptide N1/5 Aβ_{1-40/42} (mAb: "Antisenilin N1/5")

A peptide corresponding to the first five amino acid residues of Aβ (1-40 and 1-42), as schematically represented by the appropriate line segment in Fig. 2, is synthesized. The peptide contains a cysteine residue at the C terminus and has the sequence of SEQ ID NO:2 (See Figure 1) .

### Peptide N1/7Aβ_{1-40/42} (mAb: "Antisenilin N1/7")

A peptide corresponding to the first seven amino acid residues of Aβ (1-40 and 1-42), as schematically represented by the appropriate line segment in Fig. 2, are synthesized. The peptide contains a cysteine residue at the C terminus and has the sequence of SEQ ID NO:3.

### Peptide C34/40Aβ₁₋₄₀ (mAb: "Antisenilin C34/40")

A peptide corresponding to the last seven amino acid residues of Aβ (1-40), as schematically represented by the appropriate line segment in Fig. 2, is synthesized. The peptide contains a cysteine residue at the N-terminus and has the sequence of SEQ ID NO:4.

### Peptide C36/42Aβ₁₋₄₂ (MAb: "Antisenilin C36/42")

A peptide corresponding to the last seven amino acid residues of Aβ (1-42), as schematically represented by the appropriate line segment in Fig. 2, is synthesized. The peptide contains a cysteine residue at the N-terminus and has the sequence of SEQ ID NO:5.

### Peptide conjugation

The purified peptides are conjugated to bovine serum albumin (BSA) using N-maleimido-6-aminocaproyl ester of 1-hydroxyl-2-nitro-4-benzene-sulfonic acid.

### Immunization and hybridoma monoclonal antibody production

**Phase 1:** Four sets of 10 Balb/c mice are immunized with the purified BSA-conjugated peptides described above using standard immunization protocols (Taggert and Samloff, 1983). **Phase 2:** Following the completion of the immunization protocol, a fusion procedure is performed using spleenoxytes from the hyperimmunized mice and an appropriate myeloma cell-line SP2/0-Ag14 (ATCC CRL 1581), NS-1 (ATCC TIB18), or equivalent. This procedure is performed using polyethylene glycol, and the selection of successful fusion products are achieved by means of HAT media. Viable hybridoma colonies are grown out in 96 well plates.

**Phase 3:** Screening of all wells containing successful fusion products are carried out using ELISA described in the next section with the peptide antigens. Supernatants from several wells are also screened in the in vitro bioassays as described below.

**Phase 4:** On the basis of the results of ELISA assays and the evaluations based the results of the bioassays, subcloning is performed by limiting dilutions on the selected colonies.

### ELISA detection and affinity determinations

The specificity and binding affinities (Kds) of the monoclonal antibodies are evaluated by ELISA assays (Engvall and Perlmann, 1971) using a set of synthetic peptides corresponding to Aβ 1-42, Aβ 1-40, and residues 1-52, 1-11, - 2[KM]-11, -1[M]-11, 1-28, 35-40, 35-42, and 35-44 found in Aβ peptides and βAPP from which they are derived. In addition, the immunogenic peptide sequences, corresponding to the N-terminus or C-terminus of Aβ peptides, and conjugated to a different carrier protein, such as keyhole limpet hemocyanin (KLH) and ovalbumin, are used to determine whether the resultant monoclonal antibodies are end-specific for Aβ peptides and non-specific for the carrier protein or the cysteine bridge.

To test the protocol to be used subsequently to generate monoclonal antibodies, high affinity polyclonal antibodies specific for the free N-terminus of Aβ peptides were made where the antibodies were raised using the restricted peptide: H₂N - SEQ ID NO:6 - aminohexanoate-C-amide. The peptides were synthesized using solid phase Fmoc chemistry. The peptides were then cleaved and analyzed by mass spectroscopy and high performance liquid chromatography (HPLC). HPLC purification was achieved using a C-18 YMC column (10 µ packing, 120 A pore size, 10 X 250 mm) in a buffer system of A: H₂O/0.1% TFA and B:CH₃CN/0.08% TFA. The appropriate fractions were pooled, lyophilized, and again subjected to mass spectroscopy and HPLC analysis. The peptide was coupled to KLH for immunization, BSA for ELISA detection, with the cross-linker MBS. Rabbits were immunized at 3 week intervals, and the titer assessed by ELISA using acetal-SEQ ID NO:7-Ahx-C-amide. This peptide corresponds to a sequence of amino acid residues that spans the 0 to 1 splice site that yields the free N-terminus of Aβ peptides. The same spanning peptide was coupled to a thiol coupling gel via their cysteine residue and used to preabsorb away all antibodies which do not depend upon the free amine-Asp being present. The antibodies were then purified and collected using the N-terminal peptide. Whereas the crude serum shows substantial activity towards the spanning peptide, once affinity purified, there is no reactivity of the resulting antibody with the spanning peptide, only with the N-terminal peptide.

To generate monoclonal antibodies specific for the N-terminus of the Aβ peptides, mice are immunized at 3 week intervals using: H₂N-SEQ ID NO:6-aminohexanoate-C-amide conjugated to BSA prepared as described for the preparation of polyclonal. The titer in each mouse is also assessed by ELISA as described above. After spleen cell fusion of the mice containing the highest titer, several clones are isolated and screened using the spanning peptide ELISA detection method.

### In vitro bioassays to test efficacy of Aβ end-specific antibodies in blocking Aβ aggregation and Aβ-induced cytotoxicity

**A) Effects on Aβ Fibril Formation:** As shown by Jarrett et al. (1993), the carboxyterminus of Aβ is critical for the "seeding" of amyloid formation which is probably responsible for the greatly accelerated rate of amyloid plaque formation in Alzheimer's Disease (Yankner and Mesulam, 1991). Amyloid formation by the kinetically soluble peptides, such as Aβ 1-40, can be nucleated or "seeded" by peptides such as Aβ 1-42 that include the critical C-terminal residues 41(Ile) and 42(Ala). After the April 9, 1997, filing date of the provisional U.S. application on which the present application claims benefit of priority, abstracts by Solomon et al. (1997) and Frenkel et al. (1997) reported that their studies show that antibodies directed towards the N-terminal region of positions 1-16 of Aβ peptides bind to formed fibrils and lead to disaggregation. The anti-aggregating epitope of such an antibody is reported to be located within just the four amino acids Glu Phe Arg His (SEQ ID NO:8) of residue positions 3-6. These four amino acid residues of SEQ ID NO:8 are all present in the immunizing peptides for the N-terminus of Aβ. The ability of C-terminus or N-terminus end-specific Aβ antibodies to block seeding by Aβ 1-42 or to prevent aggregation of amyloid peptides is tested using standard aggregation assays (Wood et al., 1996). The Aβ 1-40 peptide is solubilized to 5 mg/ml in 1,1,1,3,3,3-hexafluoro-2-propanol. The peptide is concentrated to dryness and resolubilized in phosphate-buffered saline (PBS), pH 7.4, to a final concentration of 230 µM. A solution of Aβ 1-42 (20 µM) is stirred for 3 days and sonicated for 30 min to produce amyloid fibrils. Preaggregated Aβ1-42 at 2nM concentration is added to the supersaturated pH 7.4 incubation to seed aggregation of Aβ 1-40. Aggregate formation in the absence and in the presence of each Aβ end-specific monoclonal antibody is determined by monitoring the turbidity of samples prepared in microtiter wells using a microtiter plate reader at 405 nm. The reaction is also monitored by thioflavin-T fluorescence as described by Wood et al. (1996). The ability of N-terminus-specific antibodies to promote disaggregation of amyloid peptide fibrils is tested by testing the displacement of [¹²⁵I]-labeled amyloid aggregated peptides from a collagen matrix containing non-aggregated peptides coated onto 96-well microtiter-plastic-coated plates. In addition, the ability of N-terminus-specific-antibodies to protect neurons from Aβ-induced damage is assessed by the trypan blue exclusion method, intracellular calcium measurements, scanning and transmission electron microscopy and by confocal microscopy.

**B) Aβ-induced neurotoxicity:** The receptor for advanced glycation end products (RAGE) mediates some of the neurotoxic effects of Aβ on neurons and microglia (Yan et al., 1996). End-specific antibodies are tested for their ability to inhibit the receptor-mediated neurotoxicity by competitive inhibition. The antibodies are tested both with purified RAGE receptor preparations and by measuring their effect on Aβ-induced cellular oxidant stress.

The RAGE receptor is purified from a bovine lung extract dissolved in tris-buffered saline containing octyl-β-glucoside (1%) and phenylmethylsulfonylfluoride (2 nm) and applied to a heparin hyperD column (Biosepra). The column is eluted with a gradient of NaCl and fractions with maximal binding of ¹²⁵I-labeled Aβ are identified. The fractions are pooled and loaded onto hydroxyapatite ultragel (Biosepra) and eluted with increasing concentrations of phosphate. Fractions with maximal binding of ¹²⁵I-labeled Aβ are applied to preparative non-reduced polyacrylamide SDS gels (10%). The RAGE receptor protein Mᵣ 50,000 is identified by Coommassie Blue staining and the region in adjacent lanes are cut and eluted. Competitive inhibition by the end-specific antibodies to binding of ¹²⁵I-labeled Aβ(1-40/1-42) to the RAGE receptor is determined in a number of ways: (1) different amounts (0-150 µg) of purified protein are immobilized on microtiter wells and incubated with 100 nM ¹²⁵I-labeled Aβ(1-40/1-42); (2) different amounts (0-250 nM) of ¹²⁵I-labeled Aβ(1-40/1-42) are incubated in microtiter wells pre-coated with 5 µg purified RAGE receptor; and (3) different amounts (0-500 ug/ml) of Aβ(1-40/1-42) are immobilized on microtiter wells and incubated with 50 nM ¹²⁵I-labeled RAGE receptor. In each assay, the amount of ligand binding to the well in the presence of different amounts of antibody is determined by counting the amount of radioactivity in the wells with a gamma-scintillation counter.

To evaluate the efficacy of the different end-specific Aβ monoclonal antibodies as inhibitors of Aβ-induced cellular oxidant stress, cultured mouse brain microvascular endothelial cells (Breitner et al., 1994) are incubated with 0.25 µM Aβ in the presence of different amounts of the antibodies, and cellular oxidant stress is assessed by measuring the dose-dependent generation of thiobarbituric acid-reactive substances using the TBARS assay as previously described (Dennery et al., 1990; Yan et al., 1996). In a parallel assay system (developed by Khoury et al., 1996), the inhibitory effects of the antibodies are tested on Aβ-induced production of oxygen-reactive species in N9 mouse microglial cells. N9 cells (5 x 10⁴⁾ are incubated at 37°C in the presence of different amounts of the antibodies in 50 µl PD-BSA (phosphate-buffered saline lacking divalent cation having 1 mg/ml BSA) containing 1 µM H₂DCF (2',7'-dichlorofluorescein diacetate), a dye that fluoresces upon oxidation (Wan et al., 1993) on multispot slides coated with Aβ peptides. At various time points, aliquots of the culture medium are taken and the fluorescence is measured in a fluorescence plate reader (Cytofluor II).

**C)** **Effect on interactions with proteoglycans**: The vascular cell derived heparan sulfate proteoglycan, perlecan, has been identified in all amyloid deposits and is implicated in the earliest stages of inflammation-associated amyloid induction through high-affinity binding interactions with Aβ (Snow et al. 1989; 1995). Binding of perlecan to Aβ imparts secondary and tertiary amyloid structural features which suggest that molecules that interfere with the interaction may prevent or arrest amyloidogenesis.

End-specific Aβ monoclonal antibodies made to peptides of different lengths that correspond to the N-terminus of the peptide are evaluated for their ability to block the binding of perlecan to the perlecan binding site in the N-terminus region of Aβ (Snow et al., 1995). These evaluations are based on a solid-phase binding assay using perlecan isolated from cultured endothelial cells prepared from calf thoracic aortas as described in detail by (Snow et al. 1995). Polyvinyl micro-titer wells are coated with 100 µl of nitrocellulose solution and allowed to dry. Wells are then coated overnight at room temp with unlabeled perlecan to give 0.28 ug of bound perlecan per well, and blocked overnight at room temp with 200 µl of 5% non-fat dried milk. Various quantities of ¹²⁵I Aβ (7000 cpm/pM) diluted in 100 µl of TBS/0.05% Tween 20 (TBST) are added in triplicate to the wells and incubated for 2.5 h at room temp on an orbital shaker. At the end of the incubation period, free ¹²⁵I Aβ is removed with six washes of TEST. Bound ¹²⁵I is extracted in 100 µl 1N sodium hydroxide and "bound" versus "free" radioactivity is quantitated by liquid scintillation counting. Scatchard analysis is performed after incubating ¹²⁵I-Aβ in the presence of increasing amounts of monoclonal antibody.

### CLONING AND ASSEMBLY OF RECOMBINANT GENES

### mRNA isolation and cDNA synthesis from hybridomas

Messenger RNA (mRNA) is prepared from 5 x 10⁸ hybridoma cells as described by Griffiths and Milstein (1985). First-strand cDNA synthesis is performed according to standard procedures (Maniatis et al., 1989).

### PCR amplification, cloning of variable antigen-binding region and construction of single-chain antibodies

Techniques have been developed for the cloning of immunoglobulin variable domains from genomic DNA and cDNA using the polymerase chain reaction (Orlandi et al., 1989; Ward et al., 1989; Richardson et al., 1995). Primers based on conserved sequences at each end of the nucleotide sequences encoding V domains of mouse immunoglobulin heavy-chain (V_{H}) and kappa light-chain (V_{K}) also incorporate restriction sites that permit force-cloning of the amplified product containing the variable region of each chain. These primers are capable of amplifying most immunoglobulin mRNA of the mouse repertoire.

As shown in Fig. 4, PCR on cDNA from the hybridoma cells is performed using the primers described by Richardson et al., 1995. The scFv antisenilin gene is assembled from the amplified DNA corresponding to the V_{H} and V_{L} regions and an interchain linker, expressed in E. coli, and reamplified by PCR using primers that incorporates a stop codon at the 3'-end of V_{L} as described by Richardson et al. 1995. To prepare for the construction of a recombinant AAV vector, an XbaI restriction site is incorporated into the forward and reverse primers for reamplifying the recombinant scFv gene so as to facilitate its insertion into the AAV plasmid vector pSSV discussed immediately below.

### Construction of recombinant adeno associated viral vectors for regional expression of scFvαAβ genes in the brain

The assembled ScFvαAβ genes are ligated into an AAV plasmid pSSV9 (psub201) under the control of the human βAPP promoter (huβAPPP). Plasmid pSSV9 is a modified full-length AAV type 2 genomic clone. Alternatively, other suitable promoters, such as Thy-1, synapsin I, prion, etc. can be used as discussed previously, although huβAPPP is preferred.

As shown in Figure 5, all of the AAV coding sequences are excised, leaving only the viral inverted terminal repeats (ITR) by cleavage of the two flanking XbaI sites. These ITRs contain the recognition signals necessary for replication and packaging into an AAV vector. The AAV coding sequences are replaced with the hu βAPPP_{H/K}Aβ coding sequences. The new coding sequences of AAV/huβAPPP_{H/K}Aβ are followed by an SV40 early region polyadenylation signal.

### Preparation of packaged hu βAPPP_{H/K}AβAAV vectors

huβAPPP_{H/K}Aβ AAV vectors are packaged by co-complementation as described by Samulski et al., 1989, using an adenovirus-transformed human embryonic kidney cell line, 293 (ATCC CRL-1573). The cells are cultured in Eagle's MEM supplemented with glutamine and Earle's salts and 10% heat-inactivated fetal calf serum at 37°C in a humidified incubator with 5% CO₂. Adenovirus type 5 (Ad5) stocks are raised by 1 h infection of subconfluent 293 cells with 10 µl of Ad5 seed culture in 1 ml of serum-free DMEM per 100 mm dish. After 48 to 72 h, when strong cytopathic effects are observed, the cells are collected and pelleted by centrifugation. The cells are lysed by freeze/thaw cycles to release the intracellular virus and debris is removed by low-speed centrifugation. virus containing supernatants are aliquoted and stored -160C. Co-complementation is accomplished as follows: Semi-confluent 293 cells plated at 0.5 x 10⁶ cells per 100 mm plate are infected with Ad5 at a multiplicity of infection of 10. After 1 h, the cells are co-transfected with 20 µg of huβAPPP_{H/K}αAβ AAV plasmids and 10 µg of pAd8 which contains the AAV 2 genes encoding the AAV replication and encapsidation functions, but flanked by terminal repeats that are derived from adenovirus 2, rather than AAV (Samulski et al., 1987, 1989). Transfection is performed using a standard calcium phosphate precipitation method (Wigler et al., 1979) with the addition of chloroquine diphosphate to enhance the transfection efficiency (Luthman et al., 1983). After overnight incubation, the transfection solution is replaced with fresh medium containing 15% fetal calf serum. Three days after infection the cells are harvested, pelleted by centrifugation at 1000 x g for 10 min, and then subjected to freeze thaw cycles to release the cell-associated virons. Contaminating Ad5 is inactivated by heating lysates to 56°C. The lysates are then clarified by centrifugation and treated with 25 units/ml of RNASE-free DNASE at 37°C for 30 min to remove any remaining plasmid DNA.

### Transduction of human neurons to test expression of AAV/huβAPPP_{H/K}Aβ vectors

The utility of AAV as a potential vector has been established unequivocally by Du et al., 1996, in human NT neurons (Pleasure et al., 1993). The precursor of these neurons is a subline of human teratocarcinoma cells, NT2, that commits to terminal differentiation into neurons on exposure to retinoic acid (Lee et al., 1994; Pleasure et al., 1993). Four weeks of retinoic acid treatment accompanied by selective replatings can yield nearly pure >95 % populations of neurons. These mature neurons remain viable in culture for many weeks. In addition to the distinct morphological appearance and expression of many neuronal markers, human NT neurons have similar patterns of amyloid precursor proteins as native CNS neurons and produce Aβ peptides (Wertkin et al., 1993).

Undifferentiated precursor NT2 cells are obtained from Stratagene, La Jolla, CA, and cultured in Opti-MEM (GIBCO BRL, Gaithersburg, MD) containing 5% heat-inactivated fetal bovine serum (FBS) and 100 units/ml penicillin and 100 µg/ml streptomycin (PS) at 37°C in 5% C0₂. 2 x 10⁶ NT2 cells per T75 flask are treated with 10 µM retinoic acid for four weeks and then replated at low density into six T75 flasks. The top layers containing differentiated cells are mechanically dislodged and replated at 1 x 10⁶ cells per well in 24-well plates. Wells and glass cover-slips are coated with 0.01% poly-D-lysine followed by 1:20 MATRIGEL (Collaborative Research, Bedford, MA). Cells are cultured in DMEM high glucose/L-glutamine containing 10% FBS, PS and mitotic inhibitors for three weeks. The enriched neurons are maintained in DMEM/10% FBS/PS at 37°C, 5% CO₂.

NT neurons (about 10⁵ per well) are transduced with AAV/huβAPPP_{H/K}Aβ vectors by removing the growth medium, washing once with serum-free medium and adding vector stock diluted in serum-free DMEM. After incubating for 90 min at 37°C, 1 ml of DMEM containing 10% FBS is added to each well. The cultures receive a change of medium after two days, and twice weekly thereafter.

### Cell viability assay

The viability of cells is evaluated on the basis of the mitochondrial function of control and huβAPPPV_{H/K}Aβ AAV vector transduced cells. The levels of mitochondrial dehydrogenase activity were compared using 3-(4,5-Dimethylthiazol-3-yl)-2,5-diphenyl tetrazolium bromide as the substrate. Its cleavage to a purple formazan product by dehydrogenase is spectophotometrically quantified at 570 nm.

### Detection and determination of binding affinity constants

To verify that the secreted recombinant antibodies (antisenilins) retain the binding properties of the original hybridoma secreted antibodies, ELISA assays are performed, as described earlier in this example, with the culture medium of NT2 transduced cells.

### Bioassays to test inhibition of Aβ functions

The secreted antibodies are isolated from the culture medium in which the transduced NT2 cells are incubated. The purified antibodies and the culture-medium itself are tested as inhibitors of Aβ aggregation or Aβ induced cytotoxicity as described above in the section on in vitro bioassays.

### Generation of transgenic mice expressing the single-chain antisenilin antibodies in the brain

The ScFv antisenilin gene is inserted into a hamster prion protein (PrP) cosmid vector in which the PrP open reading frame (ORF) is replaced with the antisenilin gene ORF. The transgenes are used to generate transgenic mice by microinjection into fertilized 1-cell eggs of C57B6SJL mice according to any one of the widely used methods, such as Brinster et al. (1981), Harbers et al. (1981), Wagner et al. (1981), Gordon et al. (1976), Stewart et al. (1982), Palmiter et al. (1983), and U.S. Patent No. 4,870,009. The resulting progeny (TGScFvA) are tested by genotyping using standard PCR amplification procedures.

### Animal models to establish the therapeutic potential of the αβ antibodies as antisenilins

Animal models are required to test for the expression of anti-Aβ antibodies *in vivo* and to determine whether they demonstrate a potential for slowing down the accumulation of amyloid plaques and prevent the development of AD-like pathology in the brain. Although AD is a uniquely human disease, a number of transgenic mice that overexpress human βAPP show promise.

### Effects of chronic Aβ depletion on plaque burden and related AD pathology transgenic mice

The antisenilin function of the recombinant Aβ end-specific antibodies are tested in a transgenic animal mouse model that overexpresses the 695-amino acid isoform of Alzheimer βAPP containing a Lys670 to Asn, Met671 to Leu mutation (Hsiao, K., 1996, U.S. Patent Application no. 08/664,872). The correlative appearance of behavioral, biochemical, and pathological abnormalities reminiscent of Alzheimer's Disease in these transgenic mice (TG2576) provides the opportunity to explore the usefulness of agents to slow down or prevent the Aβ-induced pathophysiology of the disease.

Female transgenic mice (TGScFvA) homozygous for the antisenilin gene are crossed with breeding TG2576 males. The offspring that express both the antisenilin gene and the variant APP gene are compared with respect to behavioral, biochemical, and pathological abnormalities with TG2576 mice.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

All references cited herein, including journal articles or abstracts, published or unpublished U.S. or foreign patent applications, issued U.S. or foreign patents, or any other references, are entirely incorporated by reference herein, including all data, tables, figures, and text presented in the cited references. Additionally, the entire contents of the references cited within the references cited herein are also entirely incorporated by reference.

Reference to known method steps, conventional methods steps, known methods or conventional methods is not in any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

### REFERENCES

Andra, K. et al., Neurobiology of Aging 17:183-190 (1996).
Barrow, C.J. et al., J. Mol. Biol. 225:1075-1093 (1992).
Biocca, S. et al., Biochem. Biophys. Res. Commun. 197:422-427 (1993).
Biocca, S. et al., Trends in Cell Biol. 5:248-253 (1995).
Blacklow, N.R. et al., Am. J. Epidemiol. 88:368-378 (1968).
Breitner, J. et al., Neurology 44:227-232 (1994).
Brinster, R.L. et al., Cell 27:223 (1981).
Burdick, D. et al., J. Biol. Chem. 267:546-564 (1992).
Busciglio, J. et al., Proc. Natl. Acad. Sci. USA 90:2092-2096.
Byrne, G.W. et al., Proc. Natl. Acad. Sci. USA 86:5473-5477 (1989).
Cai, X.D. et al., Science 259:514-516 (1993).
Carter, B.J. In:Handbook of Parvoviruses, ed., Tijssen P.L. (CRC Press, Boca Raton, FL) 2, 247-284 (1990).
Cattaneo, A. et al., EMBO J. 6:2753-2758 (1987).
Chartier-Harlin, M.C. et al., Nature 353:844-846 (1991).
Citron, M. et al., Proc. Natl. Acad. Sci. USA 91:11993-11997 (1994).
Clements, A. et al., Neurosci. Lett. 161:17-20 (1993).
Constantini, F. et al., Nature 294:92 (1981).
Dennery, P. et al., Am. J. Resp. Cell Mol. Biol. 3:137-144 (1990).
Du, B. et al., Gene Therapy 3:254-261 (1996).
Dickson, D. et al., Am. J. Pathol. 132:86-101 (1988).
Duan, L. et al., Proc. Natl. Acad. Sci. USA 91:5075-5079 (1994).
Engvall et al., Immunochemistry 8:871-4 (1971).
Esch, F.S. et al., Science 248:1122-1124 (1990).
Fabian, H. et al., Eur. J. Biochem. 221:959-964 (1994).
Games, D. et al., Nature 373:523-527 (1995).
Glenner, G.G. et al., Biochem. Biophys. Res. Commun. 120:885-890 (1984).
Goate, A. et al., Nature 349:704-706 (1991).
Golde, T.E. et al., Science 255 (1992).
Gordon, J.W. et al., Proc. Natl. Acad. Sci. (U.S.A.) 73:1260 (1976).
Gravina SA. et al., J. Biol. Chem. *270:(13):7013-6 (1995).
Griffiths, G. and Milstein C., In: Hybridoma Technology in Biosciences and Medicine, ed. Springer, T.A. (Plenum New York). pp 103-105 (1985) .
Harbers, K. et al., Nature 293:540 (1981).
Harrington C.R. et al., Biochim. Biophys. Acta 1158 (2):120-8 (1993).
Haass, C. et al., J. Biol. Chem. 270:6186-6192 (1995).
Haass, C. et al., Nature 359:322-325 (1992).
Halverson, K. et al., Biochemistry 29:2639-2664 (1990).
Hardy, J.A. et al., Science 256:184-185 (1992).
Hendriks, L. et al., Nat. Genet. 1:218-221 (1992).
Higaki, J. et al., Neuron 14:651-659 (1995).
Higgins, L. S. et al., Ann. Neurol. 35:598-607 (1994).
Howland, D.S. et al., Neurobiology of Aging 16:685-699 (1995).
Hsiao, K., et al., Science 274:99-101 (1996).
Iwatsubo T. et al., Neuron 13. (1):45-53 (1994).
Jarrett, J.T. et al., Biochemistry 32:4693-4697 (1993).
Jarrett, J.T. et al., Cell 73:1055-1058 (1993).
Joachim, C.L. et al., Nature 341:226-230 (1989).
Joachim, C.L. et al., Am. J. Pathol. 135:309-319 (1989).
Kang et al. Nature 325:733-736 (1987).
Kaplitt, et al., Proc. Natl. Acad. Sci. USA 91:8970-8983 (1994).
Karger, B.D. FOCUS 11:57 (1989).
Khoury, J.E. et al., Nature 382:716-719 (1996).
Kiselevsky, R. et al., Nature Med. 1:143-148 (1995).
Kitaguchi, N. et al., Nature 331:530-532 (1988).
Knops, J. et al., J. Biol. Chem. 270:2419-2422 (1995).
Kohler and Milstein, Nature 256:495-4967 (1975).
Konig G. et al., J. Biol. Chem. 267:10804-10809 (1992).
Konig G. et al., Annals of the New York Academy of Sciences 777:344-55 (1996).
Kotin R.M. et al., Genomics 10:831-834 (1991).
Kotin R.M. et al., Proc. Natl. Acad. Sci. USA 87:2211-2215.
Kotin RM. et al., EMBO J. 11:5971-5078 (1992).
Laemmli, UK., Nature 227:680-5 (1971).
La Fauci, G., et al., Biochem. Biophys. Res. Comm. 159:297-304.
Lahiri, D.K., et al., Mol. Brain Res. 32:233-240 (1995).
Laine et al., 2e Coll. Soc. Franc. Neurosci., Lyons, France (1995).
Lee, V. et al., Strat. Mol. Biol. 7:28-31 (1994).
Luthman, H. et al. Nucl. Acids Res. 11:1295-1308 (1983).
Ma, J. et al., Nature 372:92-94 (1994).
Makimura, H. et al., Soc. Neurosci. Abst. 22:1661 (1996).
Maniatis, T., Fritsch, E.F., Molecular cloning: a laboratory manual. (Cold Spring Harbor Lab. Cold Spring Harbor NY) (1989).
Marasco, W. et al., Proc. Natl. Acad. Sci. USA 90:7889-7893 (1993).
Masters, C.L. et al., Proc. Natl. Acad. Sci. USA 82:4245-4249 (1985).
Mhashilkar, A. et al., EMBO J. 14:1542-1551 (1995).
Miller, D.L. et al., Arch. Biochem. Biophys. 301:41-52 (1993).
Mullan, M. et al., Nat. Genet. 1:345-347 (1992).
Murphy GM Jr., et al. Am. J. Path. 144 (5) :1082-8 (1994).
Murrell, J. et al., Science 254:97-99 (1991).
Muzyczka, N., Curr. Top. Microbiol. Immunol. 158(97):97-129 (1992).
Neve, R.L. et al., Neuron 1:669-677 (1988).
Nishimoto, I. et al., Nature 362:75-79 (1993).
Orlandi et al., Proc Natl. Acad. Sci. USA 86:3833-3837 (1989).
Palmiter, R.D. et al., Science 222:809 (1983).
Pericak-Vance, M.A. et al., Am. J. Genet. 48:1034-1050 (1991).
Piccioli, P. et al., Proc. Natl. Acad. Sci. USA 88:5611-5615 (1991).
Piccioli, P. et al., Neuron 15:373-384 (1995).
Pleasure, S.J. et al., J. Neurosci. Res. 35, 585-602 (1993).
Ponte, P. et al., Nature 331:525-527 (1988).
Richardson, J.H. et al., Proc. Natl. Acad. Sci. USA 92:3137-3141 (1995).
Roher, A.E. et al., J. Neurochem. 61:1916-1926 (1993).
Rumble, B. et al., N. Engl. J. Med. 320:1446-1452 (1989).
Saido, T.C. et al., J. Biol. Chem. 268, 25239-25243 (1993).
Saido, T. et al., J. Biol. Chem. 269:15253-15257 (1994).
Saitoh, T. et al., In: Amyloid Protein Precursor in Development, Aging and Alzheimer's Disease, C.L. Masters, ed. (Heidelberg, Germany, Springer-Verlag (1994).
Salbaum, J.M., et al., EMBO J. 7:2807-2813 (1988).
Samulski., RJ. et al., J. Virol. 61:3096-3101 (1987).
Samulski., RJ. et al., J. Virol. 63:3822-3828 (1989).
Samulski, R.J. et al., EMBO J. 10:3941-3850 (1991).
Schellenberg, G.D. et al., Science 258:668-671 (1992).
Schellenberg, G.D. et al., Ann. Neurol. 31:223-227 (1992).
Schenk, D.B. et al., J. Med. Chem. 38:4141-4154 (1995).
Schehr, R.S. Biotechnology 12:140-144 (1994).
Schubert, D. et al., Neuron 3:689-694 (1989).
Seubert, P. et al., Nature 359:325-327 (1992).
Shoji, M. et al., Science 258:126-129 (1992).
Sisodia, S. et al., FASEB 366-369 (1995).
Sisodia, S. et al., Proc. Natl. Acad. Sci. USA 89:6075-6079 (1992).
Snow, A.D. et al., Arch. Biochem. Biophys. 320:84-95 (1995).
Steinberg, R.A. et al., The J. Biol. Chem. 256: (21):10731-10734 (1981).
Stewart, T.A. et al., Science 217:1046-1048 (1982).
Suzuki, N. et al., Science 264:1336-1340 (1994).
Taggart et al. Science 219:1228-1230 (1983).
Tagliavini, F. et al., Neurosci. Lett. 93:191-196 (1988).
Tanzi, R.E. et al., Nature 331:528-530 (1988).
Tanzi, R.E. et al., Am. J. Hum. Genet. 51:273-282 (1992).
Towbin, H. et al., Proc. Natl. Acad Sci. USA 76:4350-4354 (1979).
Van Broeckhoven, C. et al., Nature 329:153-155 (1987).
Wagner, E.F. et al., Proc. Natl. Acad. Sci. (U.S.A.) 78:5016 (1981).
Wan, C.P. et al., J. Immunol. Meth. 159:131-138 (1993).
Ward, E.S. et al., Nature 341:544-546 (1989).
Weidemann, A. et al., Cell 57:115-126 (1989).
Wertkin, A.M. et al. Proc. Natl. Acad. Sci. USA 90:9513-9517 (1993).
Wigler, M. et al. Proc. Natl. Acad. Sci. USA 76:1373-1376 (1979).
Wirak et al., EMBO J. 10:289-296.
Wisniewski, T. et al., Am. J. Pathol. 145:1030-1035 (1994).
Wisniewski, T. et al., Biochem. Biophys. Res. Commun. 179:1247-1254 (1991).
Wolozin, B.L. et al., Science 232, 648-650 (1986).
Wood, S.J. et al., J. Biol. Chem. 271:4086-4092 (1996).
Wu, P. et al., Mol. Brain Res. 24:27-33 (1994).
Wu, P. et al., Neurosci. Lett. 190:73-76 (1995).
Yan, S.D. et al., Nature 382:685-691 (1996).
Yamaguchi, H. et al., Am. J. Pathol. 135:593-597 (1989).
Yamaguchi, H. et al., Acta Neuropathol. 82:13-20 (1992).
Yan, S.D. et al., Nature 382:685-691 (1996).
Yankner, B.A. et al., N. Eng. J. Med. 325:1849-1857 (1991).
Zhu et al., Science 261:209-211 (1993).

## Claims

1. A monoclonal Aβ antibody that is end specific for a free N-terminus of an amyloid β-peptide or a free C-terminus of amyloid β-peptide Aβ1-40.

2. An end-specific anti-Aβ antibody which is generated from an immunogenic peptide incorporating either a free N-terminus or a free C-terminus of an amyloid β-peptide involved in pathogenisis of Alzheimer's disease.

3. A recombinant antibody molecule which discriminates between an Aβ peptide and the β amyloid protein precursor from which it is proteolytically derived.

4. A molecule which binds specifically to a terminus/end of an Aβ peptide to slow down or prevent the accumulation of amyloid-β peptide in the extracellular space, interstitial fluid, and/or cerebrospinal fluid.

5. A recombinant DNA molecule, containing a gene encoding a recombinant antibody end-specific for N-terminus or C-terminus of an amyloid-β peptide.

6. The antibody of claim 2, wherein an immunogen portion of the amyloid β-peptide comprises the first five amino acid residues, the first seven amino acid residues or the last seven amino acid residues of the amyloid β-peptide.

7. The antibody of any one of claims 1-3, wherein the first amino acid residue of the amyloid peptide is aspartate.

8. The antibody of any one of claims 1-3, wherein said antibody is a chimeric immunoglobulin molecule, a humanized immunoglobulin molecule, or a single chain antibody.

9. The antibody of any one of claims 1-3, wherein the antibody inhibits aggregation of the amyloid β-peptide in vitro, reduces Aβ induced neurotoxicity in a patient suffering from Alzheimer's disease, inhibits the accumulation of the amyloid β peptide in the brain of a patient suffering from Alzheimer's disease, inhibits the neurotoxic consequences of amyloid deposition in a patient suffering from Alzheimer's disease, reduces the quantity of the amyloid β-peptide in the cerebrospinal fluid of a patient suffering from Alzheimer's disease, inhibits the aggregation of Aβ peptides into senile amyloid plaques, and/or clears the Aβ peptides from the central nervous system through drainage of the extracellular space, interstitial fluid and cerebrospinal fluid into the general blood circulation.

10. The antibody of any one of claims 1-3 which does not affect the biological functions of a β-amyloid precursor protein.

11. The antibody of any one of claims 1-3, wherein the antibody interferes with the ability of Aβ peptides to seed aggregation or to interacts with other molecules that either contribute to the seeding of Aβ deposition or mediate Aβ induced cytotoxic effects.

12. The antibody of any one of claims 1-3, wherein the antibody inhibits the Aβ induced receptor-mediated neurotoxicity by competitive inhibition of the RAGE receptor.

13. A composition including a recombinant DNA molecule containing an antisenilin gene in association with means for gene delivery, wherein the composition may be for use as a medicament for preventing or inhibiting the progression of Alzheimer's Disease.

14. An ectopically expressed recombinant antibody molecule which is end-specific for the N-terminus or C-terminus of an amyloid-ß peptide.

15. The antibody of claim 14, which prevents the accumulation of amyloid- ß peptide in the extracellular space, interstitial fluid, cerebrospinal fluid and/or aggregation of the amyloid-ß peptide into amyloid deposits.
